Europäisches Patentamt

European Patent Office

Office européen des brevets

(11) Publication number: **0 109 679**
**B1**

(12) **EUROPEAN PATENT SPECIFICATION**

(45) Date of publication of patent specification: **14.08.85**

(21) Application number: **83111555.5**

(22) Date of filing: **18.11.83**

(51) Int. Cl.[4]: **C 07 C 69/614,** C 07 C 67/36, C 07 C 69/734, C 07 C 45/49, C 07 C 47/228, C 07 C 47/277

(54) **Process for the simultaneous production of phenylacetate esters and phenylacetaldehydes.**

(30) Priority: **19.11.82 JP 201994/82**

(43) Date of publication of application:
**30.05.84 Bulletin 84/22**

(45) Publication of the grant of the patent:
**14.08.85 Bulletin 85/33**

(84) Designated Contracting States:
**CH DE FR GB LI**

(56) References cited:
**EP-A-0 034 430**
**FR-A-2 208 878**
**US-A-4 060 690**

(73) Proprietor: **DENKI KAGAKU KOGYO KABUSHIKI KAISHA**
**4-1, Yuraku-cho 1-chome**
**Chiyoda-ku Tokyo (JP)**

(72) Inventor: **Ayabe, Mitsukuni**
**5-1, Asahi-cho, 3-chome**
**Machida-shi Tokyo (JP)**
Inventor: **Hirano, Hideki**
**5-1, Asahi-cho, 3-chome**
**Machida-shi Tokyo (JP)**
Inventor: **Kibayashi, Iwao**
**5-1, Asahi-cho, 3-chome**
**Machida-shi Tokyo (JP)**
Inventor: **Shimizui, Tsunehiko**
**5-1, Asahi-cho, 3-chome**
**Machida-shi Tokyo (JP)**
Inventor: **Kosai, Yoshio**
**5-1, Asahi-cho, 3-chome**
**Machida-shi Tokyo (JP)**

(74) Representative: **Schübel-Hopf, Ursula et al**
**Strehl, Schübel-Hopf, Schulz Patentanwälte**
**Widenmayerstrasse 17**
**D-8000 München 22 (DE)**

Courier Press, Leamington Spa, England.

## Description

This invention relates to a process for simultaneously producing phenylacetate esters and phenylacetaldehydes.

Phenylacetate esters are substances which are important as intermediates for pharmaceuticals, agricultural chemicals and perfumes, and phenylacetaldehydes are substances which are also important as intermediates for perfumes, pharmaceuticals, amino acids and agricultural chemicals.

Heretofore, it has been proposed to produce phenylacetate ester and phenylacetaldehyde separately by a process comprising carbonylating benzyl halide and a process comprising hydroformylating benzyl halide.

As a process for producing phenylacetate ester which comprises carbonylating benzyl halide, for example, U.S. Patent No. 3,116,306 proposed a process for producing phenylacetate ester which comprises reacting benzyl halide with carbon monoxide, alcohol and a basic substance in the presence of sodium salt of hydrocobalt tetracarbonyl as a catalyst. This process, however, shows the problem that the yields are low (25%). Later, DE—A—2,240,398 disclosed a process for producing phenylacetate ester which comprises reacting benzyl chloride with carbon monoxide and alcohol with the aid of a dicobalt octacarbonyl catalyst in a weakly alkaline medium. However, no technique for simultaneously producing phenylacetate esters and phenylacetaldehydes has been disclosed.

Further, in JA—A—27155/1980 and JA—A—53241/1980 the present applicant proposed a process for producing a carboxylate ester which comprises using an alcoholic or acetone solution of cobalt tetracarbonyl anions as a catalyst in reacting carbon monoxide with alcohol and an organic halide compound in the presence of a basic substance. However, conditions for simultaneously producing phenylacetate ester and phenylacetaldehyde in good efficiency have not yet been known.

On the other hand, a process for producing phenylacetaldehyde which comprises hydroformylating benzyl halide has already been reported in Chemical Abstracts 60, 2847 e, 1964 (Hungarian Patent No. 150412), and phenylacetaldehyde can be produced in a yield of 29% by heating carbon monoxide with hydrogen under a high pressure of 140 bar in acetone as a solvent in the presence of dicobalt octacarbonyl as a catalyst.

Later, in JA—A—31128/1980 there is described that phenylacetaldehyde could be produced in a yield of 52.2% by carrying out a reaction under a high pressure of 200 bar in acetone as a solvent in the presence of both dicobalt octacarbonyl as a catalyst and an N,N-dialkyl-substituted acid amine. Moreover, in Japanese Patent Publication No. 43455/1980, there is disclosed a process for producing phenylacetaldehyde which comprises reacting benzyl halide with carbon monoxide and hydrogen at a pressure of 200 bar in the presence of a dicobalt octacarbonyl catalyst and an N,N-disubstituted carboxylic acid amide in a two-layer medium consisting of water and a water-immiscible organic solvent under conditions in which a base co-exists. Further, according to JA—A—113727/1981, phenylacetaldehyde is obtained in a yield of 70 to 91% by carrying out a reaction at a high pressure of 100 bar by use of acetonitrile as a solvent in the presence of both dicobalt octacarbonyl and a base.

As described above, in all known processes for producing phenylacetaldehyde there is required the use of relatively expensive solvents, high pressures of 100 to 200 bar and dicobalt octacarbonyl as a catalyst.

A process for simultaneously producing phenylacetate ester and phenylacetaldehyde on an industrial scale from benzyl halide as a starting material has not yet been published.

## Summary of the invention

It is an object of this invention to provide a novel process for simultaneously producing phenylacetate esters and phenylacetaldehydes which have commercial usefulness under mild conditions in an industrially advantageous manner.

According to this invention, a process for simultaneously producing phenylacetate esters and phenylacetaldehydes by reacting a benzyl halide with carbon monoxide, hydrogen and a basic substance in the presence of a cobalt carbonyl catalyst is provided, which comprises carrying out said reaction at a temperature of 30 to 80°C and a pressure of 2 to 50 bar (kg/cm²) by using a carbon monoxide/hydrogen gas mixture having a carbon monoxide/hydrogen molar ratio of 20/1 to 1/10 in the presence of an aliphatic alcohol as a solvent in an amount of at least 0.2 times the molar amount of the benzyl halide.

## Detailed description of the invention:

According to the process of this invention, it is possible to produce simultaneously phenylacetate esters and phenylacetaldehydes in high yields under markedly moderated conditions including a pressure of below 50 kg/cm² (bar) by intentionally using an aliphatic alcohol which is inexpensive but has heretofore not been used as a solvent for the production of phenylacetaldehyde.

More particularly, the use of the aliphatic alcohol allows the reaction to proceed under markedly moderated conditions including a pressure of below 50 kg/cm² (bar) and a temperature of 30 to 80°C instead of a high pressure of above 100 kg/cm² as in the conventional processes.

Moreover, it is possible to prevent side reactions by maintaining the pH at 3 to 10 during the reaction.

Moreover, a merit of a great industrial importance is the possibility of controlled production. Namely, it

2

is possible to control a phenylacetate ester/phenylacetaldehyde formation ratio within the range of from 10/1 to 1/10 by suitably controlling the $CO/H_2$ molar ratio, the amount of the aliphatic alcohol and the pH.

Thus, when a high selectivity for phenylacetaldehyde is desired, conditions such as an increased $H_2$ partial pressure in the $CO/H_2$ gas mixture, a decreased amount of the aliphatic alcohol and maintaining the pH in a neutral or weakly acidic range are necessary, and it becomes possible to carry out the simultaneous production in high yields by suitably combining these factors. On the contrary, when a high selectivity for phenylacetate ester is desired, it is possible to suitably combine reaction conditions in a reversed manner to the above.

Though the benzyl halides which can be used in this invention are not particularly limited, the inexpensive chlorides are advantageous as the halide from an industrial viewpoint. Examples of the benzyl halides include benzyl halide, o-, m- or p-chlorobenzyl halide, o-, m- or p-methylbenzyl halide, o-, m- or p-methoxybenzyl halide, p-t-butylbenzyl halide and o-, m- or p-hydroxybenzyl halide.

When the benzyl halides exemplified above are used, it is possible to produce simultaneously phenylacetate ester and phenylacetaldehyde; o-chlorophenylacetate ester and o-chlorophenyl-acetaldhyde; m-chlorophenylacetate ester and m-chlorophenylacetaldehyde; p-chlorophenylacetate ester and p-chlorophenylacetaldehyde; o-methylphenylacetate ester and o-methylphenylacetaldehyde; m-methylphenylacetate ester and m-methylphenylacetaldehyde; p-methylphenylacetate ester and p-methylphenylacetaldehyde; o-methoxyphenylacetate ester and o-methoxyphenylacetaldehyde; m-methoxyphenylacetate ester and m-methoxyphenylacetaldehyde; p-methoxyphenylacetate ester and p-methoxyphenylacetaldehyde; p-t-butylphenylacetate ester and p-t-butylphenylacetaldehyde; o-hydroxyphenylacetate ester and o-hydroxyphenylacetaldehyde; m-hydroxyphenylacetate ester and m-hydroxyphenylacetaldehyde; and p-hydroxyphenylacetate ester and p-hydroxyphenylacetaldehyde.

The cobalt carbonyls which can be used as the catalyst in this invention usually include dicobalt carbonyl, hydrocobalt tetracarbonyl, and cobalt tetracarbonyl anion. Above all, an alcoholic or acetone solution of cobalt tetracarbonyl anion is preferred. This solution can be prepared, for example, by reacting a cobalt salt with carbon monoxide and hydrogen in an alcoholic or acetone solvent containing cobalt tetracarbonyl anion (see Japanese Patent Publication No. 32007/1982, JA—A—27845/1980).

It is preferred that the amount of the catalyst used is 1/5 to 1/100 by molar ratio with respect to benzyl halide.

Though the aliphatic alcohols which can be used as the solvent are not particularly limited, lower alcohols such as methanol, ethanol, propanol, isopropanol and butanol are preferred. Its amount is at least 0.2 molar time, preferably 1 to 8 times the molar amount of the benzyl halide. Moreover, no trouble arises even if the solvent contains a small amount of water.

It is also possible to use other inert solvents such as aliphatic saturated hydrocarbon or aromatic hydrocarbon together with the alcohol.

The basic substances which can be used include alkali metal or alkaline earth metal hydroxides, carbonates and bicarbonates; organic acid alkali metal salts and ammonium salts; ammonia; and amines. Examples of these bases are sodium hydroxide, potassium hydroxide, sodium carbonate, potassium carbonate, sodium bicarbonate, sodium acetate, ammonium acetate, ammonia, and triethylamine. An alkali metal alcoholate is expensive and has a defect that it easily reacts with benzyl chloride as the raw material, forming an ether as a by-product. Its amount is 0.5 to 1.5 times, preferably 0.8 to 1.2 times the stoichiometric amount based on benzyl halide.

Even when the carbon monoxide/hydrogen gas mixture contains a small amount of inert gas, this does not adversely affect the yield.

The carbon monoxide/hydrogen molar ratio is 20/1 to 1/10, preferably 10/1 to 1/8. The reaction pressure is 2 to 50 kg/cm$^2$ (bar), industrially preferably 2 to 30 kg/cm$^2$ (bar), particularly preferably 5 to 9.5 kg/cm$^2$ (bar).

The reaction temperature is 30 to 80°C, preferably 45 to 65°C, because an excessively high temperature causes a side reaction of phenylacetaldehyde.

The pH during the initial stage of the reaction little affects the yield, but the pH in a stationary reaction state greatly affects it. Accordingly, it is necessary that the pH be maintained at 3 to 10, preferably 4 to 8. This is not difficult in this invention because an aliphatic alcohol is used as a solvent in this invention. For example, when sodium acetate, sodium carbonate, potassium carbonate, sodium bicarbonate, potassium bicarbonate or the like is used as a base, the adjustment of the pH is easy under the above-described conditions of amounts, temperatures and pressures, so that the method of charging the base is not particularly limited. Further, when a strong base such as sodium hydroxide or an alcoholate is used, it is preferred to carry out the reaction while controlling the pH by adding the base in small portions.

The method for carrying out the reaction is not particularly limited and includes, for example, a method comprising charging a pressure vessel with all the raw materials at once and feeding thereto a carbon monoxide/hydrogen gas mixture; a method comprising charging a vessel with the raw materials except benzyl chloride and, while adding thereto benzyl chloride (including one diluted with a solvent) in portions, feeding the gas mixture; and a method comprising charging a vessel with other raw materials and, while adding a catalyst in portions, feeding the gas mixture.

Furthermore, when a base which generates carbon dioxide gas as a reaction by-product is used, the gas mixture is continuously passed so as not to lower partial pressures of carbon monoxide and hydrogen.

**0 109 679**

However, the reaction is not affected if the sum of the partial pressures of carbon monoxide and hydrogen accounts for 50% of the total pressure.

The formed phenylacetate ester and phenylacetaldehyde can be separated from the reaction mixture by a usual method such as extraction or distillation. Further, phenylacetaldehyde can also be separated by the formation of an addition salt with a bisulfite which is a method for separating an aldehyde.

This invention will now be described in more detail with reference to examples.

Example 1

A 1.5-l autoclave fitted with a stirrer and a pH meter was charged with 8.0 M of methanol, 1.0 M of sodium carbonate and 131 cc of an acetone solution of cobalt tetracarbonyl anion (containing 16 g of $Co(CO)_4$), the inside of the reactor was purged with a carbon monoxide/hydrogen (1/1) gas mixture and, while passing the gas mixture at a flow rate of 30 l/hr, 2.0 M of benzyl chloride was consecutively added in portions within 3 hours at an external temperature of 60°C and a pressure of 9.5 kg/cm²G and then the reaction was continued for two additional hours.

The pH was 8.3 at the start of the reaction, lowered to 6 after 15 minutes and then was maintained at about 6 to 5.

After the reaction, the reaction solution was analyzed by gas chromatography and it was found that 92.1% of the benzyl chloride had reacted, the selectivity for methyl phenylacetate was 11.7%, and that for phenylacetaldehyde was 84.7% (methyl phenylacetate/phenylacetaldehyde=1/7.23).

Example 2

This example was carried out in the same way except that a carbon monoxide/hydrogen/nitrogen (3/1/0.2) gas mixture was used.

Analysis of the reaction solution after the reaction revealed that 91.3% of the benzyl chloride had reacted, the selectivity for methyl phenylacetate was 62.2%, and that for phenylacetaldehyde was 32.3%. The pH was 8.4 at the start of the reaction and 7.2 to 6.9 during the reaction (methyl phenylacetate/phenylacetaldehyde=1.92/1).

Example 3

The reaction of this example was carried out in the same way as in Example 1, except that the amount of methanol used was decreased to 5.0 M. The pH was 8.7 at the start of the reaction, decreased to 7.5 after 20 minutes and then was maintained at 7.1 to 6.8.

Analysis of the reaction solution after the reaction revealed that 89.6% of the benzyl chloride had reacted, the selectivity for methyl phenylacetate was 17.4%, and that for phenylacetaldehyde was 76.8% (methyl phenylacetate/phenylacetaldehyde=1/4.41).

Example 4

The reaction of this example was carried out in the same way as in Example 1, except that a methanolic solution of sodium hydroxide (5% sodium hydroxide solution) in addition to sodium carbonate was added in portions during the reaction to maintain the pH at 7.5 to 7.8. Analysis of the reaction solution after the reaction revealed that the conversion of benzyl chloride was 86.7%, the selectivity for methyl phenylacetate was 58.2% and that for phenylacetaldehyde was 25.6% (methyl phenylacetate/phenylacetaldehyde=2.27).

Example 5

The reaction of this example was carried out in the same way as in example 1 except that water was added to the initial reaction system so that the water content of the system was 3.0%.

Analysis of the reaction solution after the reaction indicated that 90.8% of the benzyl chloride had reacted, the selectivity for methyl phenylacetate was 12.5% and that for phenylacetaldehyde was 83.4%. The pH was 8.3 at the start of the reaction and was maintained at 6.5 to 5.0 during the reaction (methyl phenylacetate/phenylacetaldehyde=1/6.67).

Example 6

A 1.5-l autoclave fitted with a stirrer was charged with 2.0 M of benzyl chloride, 10 M of methanol, 0.95 M of sodium carbonate and 110 cc of a methanolic solution of cobalt tetracarbonyl anion (containing 11 g of $Co(CO)_4$). The inside of the reactor was purged with a carbon monoxide/hydrogen (1/4) gas mixture and then this gas was passed at a flow rate of 20 l/hr at a temperature of 65°C and a pressure of 25 kg/cm² G. The reaction was continued for 4 hours. Analysis of the reaction solution revealed that the conversion of benzyl chloride was 94.6%, the selectivity for methyl phenylacetate was 18.5% and that for phenylacetaldehyde was 73.2%. The pH was 7.1 to 6.9 during the reaction (methyl phenylacetate/phenylacetaldehyde=1/3.96).

Example 7

The reaction of this example was carried out in the same way as in Example 2, except that the amount of methanol used was 5.0 M. Analysis of the reaction solution after the reaction indicated that the conversion of benzyl chloride was 90.6%, the selectivity for methyl phenylacetate was 57.1%, and that for

4

phenylacetaldehyde was 34.6%. The pH was 7.1 to 6.5 during the reaction (methyl phenylacetate/phenylacetaldehyde=1.65/1).

Example 8

The reaction of this example was carried out in the same way as in Example 1, except that the temperature was 65°C and that 5 M of isopropyl alcohol as the alcohol and 2.0 M of sodium acetate were used. Analysis of the reaction solution after the reaction showed that 93.2% of the benzyl chloride had reacted, the selectivity for isopropyl phenylacetate was 17.4%, and that for phenylacetaldehyde was 74.9%. The pH was 6.1 to 4.0 during the reaction (isopropyl phenylacetate/phenylacetaldehyde=1/4.3).

Example 9

The reaction of this example was carried out in the same way as in Example 1, except that 2.0 M of sodium hydrogencarbonate was used instead of sodium carbonate. Analysis of the reaction solution after the reaction revealed that the conversion of benzyl chloride was 87.9%, the selectivity for methyl phenylacetate was 12.1%, and that for phenylacetaldehyde was 83.8%. The pH was 6.5 to 5.4 during the reaction (methyl phenylacetate/phenylacetaldehyde=1/6.92).

Example 10

A 200-cc autoclave fitted with a stirrer was charged with 0.1 M of p-methoxybenzyl chloride, 0.05 M of sodium carbonate, 0.5 M of methanol and 3.0 g of dicobalt octacarbonyl and purged with a carbon monoxide/hydrogen (1/1) gas mixture. The reaction mixture was reacted for 6 hours at an external temperature of 60°C and a pressure of 9.5 kg/cm² while the gas mixture was being passed at a flow rate of 3 l/hr.

Analysis of the reaction solution after the reaction revealed that the conversion of p-methoxybenzyl chloride was 88.7%, the selectivity for methyl p-methoxyphenylacetate was 13.1%, and that for p-methoxyphenylacetaldehyde was 79.4%. The pH was maintained at 6.2 to 5.4 during the reaction (methyl p-methoxyphenylacetate/p-methoxyphenylacetaldehyde=1/6.01).

Example 11

The reaction of this example was carried out in the same way as in Example 10, except that 7 cc of an acetone solution of cobalt tetracarbonyl anion (containing 0.85 g of cobalt tetracarbonyl anion) was used. Analysis of the reaction solution after the reaction indicated that 91.3% of the p-methoxybenzyl chloride had reacted, the selectivity for methyl p-methoxyphenylacetate was 15.4%, and that for p-methoxyphenylacetaldehyde was 80.9%. The pH was 6.3 to 5.5 during the reaction (methyl p-methoxyphenylacetate/p-methoxyphenylaldehyde=1/5.25).

Example 12

The reaction of this example was carried out in the same way as in Example 1, except that a carbon monoxide/hydrogen (8/1) gas mixture was used as the gas. Analysis of the reaction solution after the reaction showed that 98.0% of the benzyl chloride had reacted, the selectivity for methyl phenylacetate was 88.4%, and that for phenylacetaldehyde was 9.1%. The pH was 7.5 to 7.0 during the reaction (methyl phenylacetate/phenylacetaldehyde=9.7/1).

Example 13

The reaction of this example was carried out in the same way as in Example 1, except that benzyl chloride was placed together with other raw materials, and an acetone solution of cobalt tetracarbonyl anion was added thereto in portions.

Analysis of the reaction solution after the reaction revealed that 91.9% of the benzyl chloride had reacted, the selectivity for methyl phenylacetate was 12.3%, and that for phenylacetaldehyde was 83.6%. The pH was maintained at 6.1 to 5.4 during the reaction (methyl phenylacetate/phenylacetaldehyde=1/6.80).

Example 14

This example was carried out in the same way as in Example 1, except that a reaction vessel had been charged with an acetone solution of cobalt tetracarbonyl anion, and sodium carbonate; that a benzyl chloride/methanol mixture was added in portions for one hour; and that the reaction was continued for five hours. The pH was maintained at about 6.2 to 5.5 during the reaction. Analysis of the reaction solution after the reaction showed that the conversion of benzyl chloride was 94.6%, the selectivity for methyl phenylacetate was 11.9%, and that for phenylacetaldehyde was 84.6% (methyl phenylacetate/phenylacetaldehyde=1/7.1).

Referential example

Air was passed through the reaction solution of Example 14 for 3 hours to decompose the cobalt tetracarbonyl anion, and the solvent was distilled off from the solution under a vacuum of 60 mmHg. Then, 100 g of toluene, 320 g of water and 15 g of $H_2SO_4$ were added, stirred for 15 minutes and allowed to settle.

**0 109 679**

The water phase in the lower layer was removed, then 200 cc of a 7% aqueous NaOH solution was added to the organic phase and the mixture was stirred for 5 minutes. After settlement, the water phase in the lower layer was removed and the organic phase was vacuum-distilled. A gas-chromatographic analysis of the produced organic phase revealed that the recovery of methyl phenylacetate from the reaction solution was 98% and that of phenylacetaldehyde was 85%.

Moreover, when aluminum chloride was added to the distillation residue and the residue was distilled, the recovery of phenylacetaldehyde was increased to 93%.

Further, when an aqueous solution of $NaHSO_3$ was added to the produced distillate, an aqueous solution or slurry of an $NaHSO_3$ addition salt of phenylacetaldehyde could be obtained quantitatively, and when the organic layer was further rectified, methyl phenylacetate was obtained at a recovery rate of 98%.

**Claims**

1. A process for simultaneously producing phenylacetate esters and phenylacetaldehydes by reacting a benzyl halide with carbon monoxide, hydrogen and a basic substance in the presence of a cobalt carbonyl catalyst, characterized in that said reaction is carried out at a temperature of 30 to 80°C and a pressure of 2 to 50 bar (kg/cm²) by using a carbon monoxide/hydrogen gas mixture having a carbon monoxide/hydrogen molar ratio of 20/1 to 1/10 in the presence of an aliphatic alcohol as a solvent in an amount of at least 0.2 times the molar amount of the benzyl halide.

2. A process according to claim 1, wherein an acetone or alcoholic solution of cobalt tetracarbonyl anion is used as said cobalt carbonyl catalyst.

3. A process according to either of claims 1 or 2, wherein the pH is maintained within the range of from 3 to 10 during said reaction.

4. A process according to any of the claims 1 to 3, wherein said basic substance is selected from the group consisting of alkali metal or alkaline earth metal hydroxides, carbonates, and bicarbonates; organic acid alkali metal salts and ammonium salts; ammonia; and amines.

**Patentansprüche**

1. Verfahren zur gleichzeitigen Herstellung von Phenylessigsäureestern und Phenylacetaldehyden durch Umsetzung eines Benzylhalogenids mit Kohlenmonoxyd, Wasserstoff und einer basischen Substanz in Gegenwart eines Kobaltcarbonyl-Katalysators, dadurch gekennzeichnet, daß die Reaktion bei einer Temperatur von 30 bis 80°C und einem Druck von 2 bis 50 bar (kg/cm²) unter Anwendung eines Kohlenmonoxid/Wasserstoff-Gasgemisches mit einem Molverhältnis von Kohlenmonoxid zu Wasserstoff von 20:1 zu 1:10 in Gegenwart eines aliphatischen Alkohols als Lösungsmittel in einer Menge von mindestens dem 0,2-fachen der molaren Menge des Benzylhalogenids durchgeführt wird.

2. Verfahren nach Anspruch 1, bei dem als Kobaltcarbonyl-Katalysator eine Aceton- oder alkoholische Lösung von Kobalttetracarbonyl-Anion verwendet wird.

3. Verfahren nach Anspruch 1 oder 2, bei dem der pH-Wert während dieser Reaktion innerhalb des Bereiches von 3 bis 10 gehalten wird.

4. Verfahren nach einem der Ansprüche 1 bis 3, bei dem die basische Substanz aus der Gruppe der Alkalimetall oder Erdalkalimetallhydroxide, -carbonate und -bicarbonate; der Alkalimetallsalze und Ammoniumsalze organischer Säuren; Ammoniak und der Amine ausgewählt wird.

**Revendications**

1. Procédé de production simultanée d'esters phénylacétate et de phénylacétaldéhydes, consistant à faire réagir un halogénure de benzyl avec du monoxyde de carbone, de l'hydrogène et une substance basique en présence d'un catalyseur au cobalt carbonyle, caractérisé en ce que ladite réaction est effectuée à une température de 30 à 80°C et sous une pression de 2 à 50 bar (kg/cm²), en utilisant un mélange gazeux de monoxyde de carbone/hydrogène ayant un rapport molaire monoxyde de carbone/hydrogène de 20/1 à 1/10, en présence d'un alcool aliphatique comme solvant, en une quantité d'au moins 0,2 fois la quantité molaire d'halogénure de benzyle.

2. Procédé selon la revendication 1, caractérisé en ce qu'on utilise comme catalyseur au cobalt carbonyle, une solution à l'acétone ou alcoolique d'anion cobalt tétracarbonyl.

3. Procédé selon l'une des revendications 1 ou 2, caractérisé en ce que le pH est maintenu à une valeur comprise entre 3 et 10 durant ladite réaction.

4. Procédé selon l'une des revendications 1 à 3, caractérisé en ce que ladite substance basique est choisie dans le groupe comprenant des hydroxydes, carbonates et bicarbonates de métaux alcalins ou de métaux alcalino-terreux, des sels de métaux alcalins et d'ammonium, l'ammoniaque et des amines.